# NOUVEAU FASCICULE DE BREVET EUROPEEN

(11) **EP 0 974 341 B2**
(45) Date de publication et mention de la décision concernant l'opposition: **15.03.2017**
(45) Mention de la délivrance du brevet: 01.03.2006
(21) Numéro de dépôt: 99401382.9
(22) Date de dépôt: 09.06.1999
(51) Int. Cl.: A61Q 19/10

(54) **Composition moussante conditionnante et détergente**
Schäumende und konditionierende Reinigungs-Zusammensetzung
Foaming conditioning and washing composition

(30) Priorité: 24.06.1998 FR 9808007
(43) Date de publication de la demande: 26.01.2000
(73) Titulaire: L'Oréal, 75008 Paris (FR)
(72) Inventeur: Maurin, Véronique, 75016 Paris (FR); Beauquey, Bernard, 92110 Clichy (FR); Mellul, Myriam, 94240 L'Hay les Roses (FR)
(74) Mandataire: Dossmann, Gérard

(56) Documents cités:
- EP-A- 0 589 407
- EP-A- 0 628 305
- EP-A- 0 629 396
- EP-A1- 0 727 204
- EP-A1- 0 980 683
- EP-B1- 0 801 130
- WO-A-94/03150
- WO-A-96/37285
- WO-A1-93/19149
- DE-A- 4 405 127
- DE-A- 19 511 637
- DE-C1- 4 127 731
- US-A- 4 963 535
- US-A- 5 324 507

## Description

La présente invention concerne une composition cosmétique à la fois conditionnante et détergente pour le soin et le lavage simultanés des matières kératiniques.
L'invention concerne aussi l'utilisation de ladite composition dans l'application susmentionnée.

Pour le nettoyage et/ou le lavage des cheveux et/ou de la peau, l'utilisation de compositions détergentes (shampooing ou gel-douche) à base essentiellement d'agents tensioactifs classiques de type notamment anionique, non ionique et/ou amphotère, mais plus particulièrement de type anionique, est courante. Ces compositions sont appliquées sur cheveux ou peau mouillés et la mousse générée par massage ou friction avec les mains permet, après rinçage à l'eau, l'élimination des diverses salissures initialement présentes sur les cheveux ou la peau.

Ces compositions de base possèdent certes un bon pouvoir lavant, mais les propriétés cosmétiques intrinsèques qui leur sont attachées restent toutefois assez faibles, notamment en raison du fait que le caractère relativement agressif d'un tel traitement de nettoyage peut entrainer à la longue sur les matières kératiniques des dommages plus ou moins marqués liés en particulier a l'élimination progressive des lipides ou protéines contenues dans ou à la surface de ces dernières.

Aussi, pour améliorer les propriétés cosmétiques des compositions détergentes ci-dessus, et plus particulièrement de celles qui sont appelées à être appliquées sur des cheveux sensibilisés (i.e. des cheveux qui se trouvent abîmés ou fragilisés notamment sous l'action chimique des agents atmosphériques et/ou de traitements capillaires tels que permanentes, teintures ou décolorations), il est maintenant usuel d'introduire dans ces dernières des agents cosmétiques complémentaires dits agents conditionneurs destines principalement à réparer ou limiter les effets néfastes ou indésirables induits par les différents traitements ou agressions que subissent, de manière plus ou moins répétés, les fibres capillaires. Ces agents conditionneurs peuvent bien entendu également améliorer le comportement cosmétique des cheveux naturels.

Les agents conditionneurs les plus couramment utilisés à ce jour dans des shampooings sont les polymères cationiques, les silicones et/ou les dérivés siliconés, qui confèrent en effet aux cheveux lavés, secs ou mouillés, une facilité de démêlage, une douceur et un lissage accrus par rapport à ce qui peut être obtenu avec les compositions nettoyantes correspondantes qui en sont exemptes.

Toutefois, et malgré les progrès réalisés récemment dans le domaine des shampooings à base de polymères cationiques et de silicone, ces derniers ne donnent pas vraiment complètement satisfaction, de sorte qu'un fort besoin existe encore actuellement quant à pouvoir disposer de nouveaux produits présentant, au niveau de l'une ou de plusieurs des propriétés cosmétiques évoquées ci-avant, de meilleures performances.

On a déjà proposé d'utiliser les huiles végétales ou animales en tant qu'agent conditionneur. Cependant, les compositions classiques ont des propriétés détergentes et moussantes non satisfaisantes. De plus les matières kératiniques traitées avec ces compositions présentent le plus souvent un toucher gras rédhibitoire.

DE 19511637 décrit aux exemples 3.11 et 3.12 des compositions moussantes comprenant un tensio-actif non ionique de type alkylpolyglucoside (APG), un tensioactif anionique sulfate et une huile volatile.

DE 4405127 décrit des compositions contenant un APG, un polymère et une huile végétale, non volatile.

La présente invention vise à remédier aux inconvénients cités ci-dessus en proposant des compositions conditionnantes et détergentes, suffisamment moussantes, qui présentent de bonnes propriétés de conditionnement, et notamment de démêlage, de douceur et de brillance sans conférer de caractère gras.

Ainsi, après de nombreuses recherches menées sur la question, la Demanderesse a maintenant découvert, de façon totalement inattendue et surprenante, qu'en associant une huile végétale non volatile, un tensioactif anionique de type sulfate, un agent tensioactif non ionique choisi dans le groupe des alkylpolyglycosides, il est possible d'obtenir des compositions détergentes présentant d'excellentes propriétés cosmétiques, en particulier de démêlage, de douceur et de brillance et de volume des matières kératiniques traitées et ceci tout en conservant leur bon pouvoir lavant intrinsèque et leur pouvoir moussant.

Ces nouvelles compositions permettent de déposer une quantité plus importante d'huile sur les matières kératiniques (notamment les cheveux) qu'avec une composition classique, mais sans toucher ou aspect visuel gras.

Les compositions conformes à l'invention confèrent aux matières kératiniques notamment les cheveux, un remarquable effet traitant qui se manifeste notamment par une facilité de démêlage, ainsi qu'un apport de volume, de légèreté, de lissage, de douceur et de souplesse et de discipline sans aucune sensation de gras.

La présente invention a ainsi pour objet une nouvelle composition cosmétique moussante conditionnante et détergente, qui est caractérisée par le fait qu'elle comprend, dans un milieu aqueux :
(A) au moins une huile végétale non volatile, dans des proportions de 0,2 à 20% en poids par rapport au poids total de la composition;
(B) au moins un agent tensioactif anionique de type sulfate, dans des proportions de 1 à 20% en poids par rapport au poids total de la composition ;
(C) au moins un agent tensioactif non ionique de type alkylpolyglycoside,
le rapport en poids (B)/(C) étant inférieur ou égal à 2.

La présente invention a également pour objet l'utilisation de la composition selon l'invention pour le soin et le lavage simultanés des matières kératiniques telles que les cheveux et la peau.

Une description détaillée de la présente invention va maintenant être donnée.

Selon l'invention, le rapport en poids (B)/(C) est de préférence compris entre 0,1 et 1,8, plus particulièrement entre 0,5 et 1,7 et encore plus particulièrement entre 1 et 1,5.

Les huiles végétales non volatiles pouvant être utilisées dans les compositions de l'invention sont des huiles naturelles éventuellement hydrogénées et généralement insoluble dans l'eau.
Généralement, les huiles végétales ne contiennent pas de mono ou diglycérides d'acides gras et de préférence moins de 2% en poids par rapport au poids de l'huile.

Un huile végétale non volatile, selon l'invention, est une huile qui présente une température d'ébullition généralement supérieure à 300°C sous 760 mm de Hg (101325 Pa) et qui ne présente pas ou peu de tension de vapeur.
En particulier, les huiles essentielles, qui sont des huiles volatiles ne sont pas comprises dans la définition des huiles végétales selon l'invention.

Parmi les huiles végétales, on peut citer notamment les huiles de tournesol, d'avocat, de jojoba, de maïs, d'amande douce, de soja, de courge, de pépins de raisin, de sesame, de noisette, de palme, de ricin, de noix, de noix de cajou, de Purcellin.

On préfère utiliser les huiles issues de végétaux dicotylédones telles que l'huile d'avocat et l'huile de jojoba.

Les tensioactifs anioniques de type sulfate utilisables, seuls ou mélanges, dans le cadre de la présente invention, sont les sels (en particulier sels alcalins, notamment de sodium, sels d'ammonium, sels d'amines, sels d'aminoalcools ou sels de magnésium) des alkylsulfates, alkyléthersulfates, alkylamidoéthersulfates, alkylaryléthersulfates ; les le radical alkyle de tous ces différents composés comportant de préférence de 8 à 24 atomes de carbone, et le radical aryle désignant de préférence un groupement phényle ou benzyle.
Le nombre moyen de groupements oxyde d'éthylène ou oxyde de propylène pouvant aller notamment de 2 à 50 et plus particulièrement de 2 à 10.

On préfère utiliser selon l'invention les sels d'alkylsulfates et d'alkyléthersulfates et leurs mélanges.

Parmi ces tensioactifs anioniques, on préfère utiliser les sels d'alkyléthersulfates en C₈-C₁₄ et plus particulièrement ceux en C₁₂-C₁₄. Ces sels comprennent notamment de 2 à 5 groupements d'oxyde d'éthylène. On utilise de préférence un agent tensioactif anionique choisi parmi les alkyl (C₁₂-C₁₄)éthersulfates de sodium, de triéthanolamine ou d'ammonium oxyéthylénés à environ 2,2 moles d'oxyde d'éthylène.

Le ou les agents tensioactifs non ioniques de type alkylpolyglycoside, utilisés dans le cadre de la présente invention, sont des produits bien connus en soi, et ils peuvent être plus particulièrement représentés par la formule générale (I) suivante :

R_{I}-O-(R₂O)ₜ-(G)ᵥ (I)

dans laquelle R₁ représente un radical alkyle linéaire ou ramifié, saturé ou insaturé, linéaire ou ramifié comportant environ de 8 à 24 atomes de carbone, un radical alkylphényle dont le radical alkyle linéaire ou ramifié comporte environ de 8 à 24 atomes de carbone, R₂ représente un radical alkylène comportant de 2 à 4 atomes de carbone, G représente un sucre réduit comportant de 5 à 6 atomes de carbone, t désigne une valeur allant de 0 à 10 et v désigne une valeur allant de 1 à 15.

Des alkylpolyglycosides préférés selon la présente invention sont des composés de formule (I) dans laquelle R₁ désigne plus particulièrement un radical alkyle saturé ou insaturé, linéaire ou ramifié comportant de 8 à 14 atomes de carbone, t désigne une valeur allant de 0 à 3 et plus particulièrement encore égale à 0, G désigne le glucose, le fructose ou le galactose, de préférence le glucose. Le degré de polymérisation (S) du saccharide, i.e. la valeur de v dans la formule (I), peut aller de 1 à 15. Selon l'invention, on préfère les sucres réduits contenant 80%, ou plus, de sucres dont le degré de polymérisation (S) prend une valeur allant de 1 à 4. Le degré moyen de polymérisation est plus particulièrement compris entre 1 et 2.

Des composés de formule (I) sont notamment représentés par les produits vendus par la société HENKEL sous la dénomination APG, tels que les produits APG 300, APG 350, APG 500, APG 550, APG 625, APG base 10-12, sous les dénominations PLANTAREN (1200 et 2000) ou PLANTACARE (818, 1200 et 2000). On peut également utiliser les produits vendus par la société SEPPIC sous les dénominations TRITON CG 110 (ou ORAMIX CG 110) et TRITON CG 312 (ou ORAMIX NS 10), les produits vendus par la société B.A.S.F. sous la dénomination LUTENSOL GD 70 ou encore ceux vendus par la société CHEM Y sous la dénomination AG10 LK.

Il est également possible selon l'invention d'associer aux deux types de tensioactifs décrits ci-dessus, un agent tensioactif de type amphotère.

Les agents tensioactifs amphotères, peuvent être notamment (liste non limitative):
- des dérivés d'amines secondaires ou tertiaires aliphatiques, dans lesquels le radical aliphatique est une chaine linéaire ou ramifiée comportant de 8 à 18 atomes de carbone et contenant au moins un groupe anionique hydrosolubilisant (par exemple carboxylate, sulfonate, sulfate, phosphate ou phosphonate),
- des alkyl (C₈-C₂₀) bétaïnes, des alkyl (C₈-C₂₀) sulfobétaïnes, des alkyl (C₈-C₂₀) amidoalkyl (C₁-C₆) bétaïnes ou des alkyl (C₈-C₇₀) amidoalkyl (C₁-C₆) sulfobétaïnes.

Parmi les dérivés d'amines, on peut citer les produits commercialisés sous les dénominations MIRANOL TM, tels que décrits dans les brevets US-2 528 378 et US-2 781 354 et de structures:

R₂-CONHCH₂CH₂-N(R₃)(R₄)(CH₂COO-) (2)

dans laquelle : R₂ désigne un radical alkyle dérivé d'un acide R₂-COOH présent dans l'huile de coprah hydrolysée, un radical heptyle, nonyle ou undécyle, R₃ désigne un groupement bêta-hydroxyéthyle et R₄ un groupement carboxyméthyle ; et

R_{2'}-CONHCH₂CH₂-N(D)(E) (3)

dans laquelle :
D représente -CH₂CH₂OX', E représente -(CH₂)_{z}-Y', avec z = 1 ou 2,
X' désigne le groupement -CH₂CH₂-COOH ou un atome d'hydrogène
Y' désigne -COOH ou le radical -CH₂-CHOH-SO₃H
R_{2'} désigne un radical dérivé d'acide présent dans l'huile de coprah ou dans l'huile de lin hydrolysée, un radical alkyle, notamment en C₇, C₉, C₁₁ ou C₁₃, un radical alkyle en C₁₇ et sa forme iso, un radical C₁₇ insaturé.

Ces composés sont classés dans le dictionnaire CTFA, 5^{ème} édition, 1993, sous les dénominations Disodium Cocoamphodiacetate, Disodium Lauroamphodiacetate, Disodium Caprylamphodiacetate, Disodium Capryloamphodiacetate, Disodium Cocoamphodipropionate, Disodium Lauroamphodipropionate, Disodium Caprylamphodipropionate, Disodium Capryloamphodipropionate, Lauroamphodipropionic acid, Cocoamphodipropionic acid.
A titre d'exemple on peut citer le cocoamphodiacetate commercialisé sous la dénomination commerciale MIRANOL® C2M concentré par la société RHONE POULENC.

Selon la présente invention, on préfère plus particulièrement utiliser les agents tensioactifs amphotères appartenant au groupe des bétaines tels que les alkylbétaïnes en particulier la cocoylbétaïne commercialisée sous la denomination « DEHYTON AB 30 » en solution aqueuse à 30 % de MA par la société HENKEL ou les alkylamidobétaines telles que la TEGOBETAINE® F50 commercialisée par la société GOLDSCHMIDT.

Selon l'invention, la composition peut également contenir des tensioactifs anioniques de type phosphate, sulfonate et/ou carboxylate.
A titre d'exemple, on peut citer les alkylsulfonates, alkylphosphates, alkylamidesulfonates, alkylarylsulfonates, alpha -oléfine-sulfonates, paraffine-sulfonates ; les alkylsulfosuccinates, les alkylethersulfosuccinates, les alkylamidesulfosuccinates; les alkylsulfosuccinamates ; les alkylsulfoacétates ; les alkylétherphosphates; les acylsarcosinates ; les acyliséthionates et les N-acyltaurates, le radical alkyle ou acyle de tous ces différents composés comportant de préférence de 12 à 20 atomes de carbone, et le radical aryle désignant de préférence un groupement phényle ou benzyle. On peut encore citer plus particulièrement les acides d'alkyl D galactoside uroniques et leurs sels ainsi que les acides alkyl (C₆-C₂₄) ether carboxyliques polyoxyalkylenes, les acides alkyl(C₆-C₂₄)aryl ether carboxyliques polyoxyalkylénés ,les acides alkyl(C₆-C₂₄) amido éther carboxyliques polyoxyalkylénés et leurs sels, en particulier ceux comportant de 2 à 50 groupements oxyde d'éthylène, et leurs mélanges.

Le(s) agent(s) tensioactif(s) anionique(s) de type sulfate sont présents à raison de 1 à 20% en poids, de préférence de 3 à 15% en poids, par rapport au poids total de la composition.

Le(s) agent(s) tensioactifs) non ionique(s) de type alkylpolyglycoside sont généralement présents à raison de 0,5 à 15%, de préférence de 1 à 10% en poids, par rapport au poids total de la composition.

Le(s) agent(s) tensioactif(s) amphotère(s) sont généralement présents à raison de 0,5 à environ 10% en poids, de préférence de 1 à 5% en poids, par rapport au poids total de la composition.

Lorsqu'ils sont présents, les tensioactifs amphotères peuvent représenter environ moins de 30% en poids de la totalité des tensioactifs anioniques et des alkyl polyglycosides.

Lorsqu'ils sont présents, les tensioactifs anioniques de type phosphate, sulfonate etlou carboxylate peuvent représenter environ moins de 30% en poids de la totalité des tensioactifs anioniques.

Généralement, le rapport en poids tensioactifs anioniques / alkyl polyglycosides est inférieur ou égal à 2.

Dans la composition selon la présente invention, la totalité des tensioactifs détergents représente généralement de 3 à 50% en poids et de préférence de 5 à 30% en poids par rapport au poids total de la composition.

La ou les huiles végétales non volatiles sont utilisées dans les compositions conformes à l'invention dans des proportions de 0,2 à 20 % en poids, de préférence de 1 à 8 % en poids par rapport au poids total de la composition.

Le milieu aqueux cosmétiquement acceptable peut être constitué uniquement par de l'eau ou par un mélange d'eau et d'un solvant cosmétiquement acceptable tel qu'un alcool inférieur en C₁-C₄, comme l'éthanol, l'isopropanol, le tertiobutanol, le n-butanol ; les alkylèneglycols comme le propylèneglycol, les éthers de glycols.

Les compositions détergentes selon l'invention présentent un pH final généralement compris entre 3 et 10. De préférence, ce pH est compris entre 5 et 8. L'ajustement du pH à la valeur désirée peut se faire classiquement par ajout d'une base (organique ou minérale) dans la composition, par exemple de la soude, de l'ammoniaque ou une (poly)amine primaire, secondaire ou tertiaire comme la monoéthanolamine, la diéthanolamine, la triéthanolamine, l'isopropanolamine ou la propanediamine-1,3, ou encore par ajout d'un acide, de préférence un acide carboxylique tel que par exemple l'acide citrique.

Les compositions conformes à l'invention peuvent contenir en plus de l'association définie ci-dessus des agents régulateurs de viscosité tels que des électrolytes, ou des agents épaississants. On peut citer en particulier le chlorure de sodium, le xylène sulfonate de sodium, les scléroglucanes, les gommes de xanthane, les alcanolamides d'acide gras, les alcanolamides d'acide alkyl éther carboxylique éventuellement oxyéthylénés avec jusqu'à 5 moles d'oxyde d'éthylène tel que le produit commercialisé sous la dénomination « AMINOL A15» par la société CHEM Y, les acides polyacryliques réticulés et les copolymères acide acrylique / acrylates d'alkyle en C₁₀-C₃₀ reticules. Ces agents régulateurs de viscosité sont utilisés dans les compositions selon l'invention dans des proportions pouvant aller jusqu'à 10% en poids par rapport au poids total de la composition.

Les compositions conformes à l'invention peuvent également contenir jusqu'à 5% d'agents nacrants ou opacifiants bien connus dans l'etat de la technique tels que par exemple les palmitates de sodium ou de magnésium, les stéarates et hydroxystéarates de sodium ou de magnesium, les dérivés acylés à chaîne grasse tels que les monostéarates ou distéarates d'éthylène glycol ou de polyéthylèneglycol, les alcools gras, les éthers à chaînes grasses tels que par exemple le distéaryléther ou le 1-(hexadécyloxy)-2-octadécanol.

Les compositions conformes à l'invention peuvent éventuellement contenir en outre d'autres agents ayant pour effet d'améliorer les propriétés cosmétiques de cheveux ou de la peau sans cependant altérer la stabilité et/ou les propriétés lavantes et moussantes des compositions. On peut citer à ce sujet les agents tensioactifs cationiques, les polymeres anioniques ou non ioniques ou cationiques ou amphotères, les protéines, les hydrolysats de protéines, les céramides, les pseudocéramides, les acides gras à chaines linéaires ou ramifiées en C₁₆-C₄₀ tels que l'acide méthyl-18 eicosanoique, les hydroxyacides, les vitamines, le panthénol, les esters d'acides gras, les silicones volatiles ou non volatiles, solubles et insolubles dans le milieu, les agents hydratants, les agents antipelliculaires ou antiséborrhéiques, les filtres solaires, les agents anti-radicaux libres, les huiles minérales, les huiles organiques de synthèse et leurs mélanges.

La quantité totale de composés lipophiles tels que par exemple les huiles végétales selon l'invention, les silicones, les huiles minérales est généralement inférieure à 20% en poids par rapport au poids total de la composition.

Les polymères cationiques utilisables conformément à la présente invention peuvent être choisis parmi tous ceux déjà connus en soi comme améliorant les propriétés cosmétiques des cheveux traités par des compositions détergentes, à savoir notamment ceux décrits dans la demande de brevet EP-A-0 337 354 et dans les demandes de brevets français FR-A-2 270 846, 2 383 660, 2 598 611, 2 470 596 et 2 519 863.

De manière encore plus générale, au sens de la présente invention, l'expression « polymère cationique » désigne tout polymère contenant des groupements cationiques et/ou des groupements ionisables en groupements cationiques.

Parmi tous les polymères cationiques susceptibles d'être utilisés dans le cadre de la presente invention, on préfère mettre en oeuvre les dérivés d'éther de cellulose quaternaires tels que les produits commercialisés sous la dénomination « JR 400 » par la société UNION CARBIDE CORPORATION, les cyclopolymères, en particulier les homopolymères de sel de diallyldiméthylammonium et les copolymères de sel de diallyldiméthylammonium et d'acrylamide en particulier les chlorures, commercialisés sous les dénominations « MERQUAT 100 », « MERQUAT 550 » et « MERQUAT S » par la société MERCK, les polysaccharides cationiques et plus particulièrement les gommes de guar modifiées par du chlorure de 2,3-epoxypropyl triméthylammonium commercialisées par exemple sous la denomination « JAGUAR C13S » par la société MEYHALL, les homopolymères et les copolymères éventuellement réticulés de sel de (méth)acryloyloxyéthyltriméthylammonium, vendus par la société ALLIED COLLOIDS en solution à 50% dans de l'huile minérale sous les dénominations commerciales SALCARE SC92 (copolymère réticulé du chlorure de méthacryloyloxyéthyltriméthylammonium et de l'acrylamide) et SALCARE SC95 (homopolymère réticulé du chlorure de méthacryloyloxyéthyl triméthylammonium).
On peut également utiliser les polymères qui sont constitués de motifs récurrents répondant à la formule : 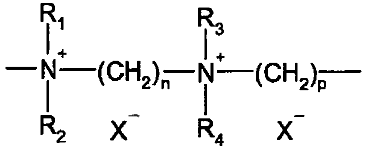 dans laquelle R₁, R₂, R₃ et R₄, identiques ou différents, désignent un radical alkyle ou hydroxyalkyle ayant de 1 à 4 atomes de carbone environ, n et p sont des nombres entiers variant de 2 à 20 environ et, X⁻ est un anion dérivé d'un acide minéral ou organique.

Un composé de formule (a) particulièrement préféré est celui pour lequel R₁, R₂, R₃ et R₄, représentent un radical méthyle et n = 3, p = 6 et X = Cl, dénommé Hexadimethrine chloride selon la nomenclature INCI (CTFA).

Selon l'invention, le ou les polymères cationiques peuvent représenter de 0,001 % à 10% en poids, de préférence de 0,005% à 5% en poids, et encore plus préférentiellement de 0,01% à 3% en poids, du poids total de la composition finale.

Les compositions selon l'invention peuvent contenir également des synergistes de mousses tels que des 1,2-alcanediols en C₁₀-C₁₈ ou des alcanolamides gras dérivés de mono ou de diéthanolamine.

Bien entendu, l'homme du métier veillera à choisir ce ou ces éventuels composés complémentaires et/ou leurs quantités de manière telle que les propriétés avantageuses attachées intrinsèquement à l'association conforme à l'invention ne soient pas, ou substantiellement pas, altérées par la ou les adjonctions envisagées.

Le pouvoir moussant des compositions selon l'invention, caractérisé par une hauteur de mousse, est généralement supérieur à 75 mm ; de préférence, supérieure à 100 mm mesurée selon la méthode ROSS-MILES (NF T 73-404 /ISO696) modifiée.
Les modifications de la méthode sont les suivantes :
La mesure se fait à la température de 22°C avec de l'eau osmosée. La concentration de la solution est de 2g/L. La hauteur de la chute est de 1 m. La quantité de composition qui chute est de 200 ml. Ces 200 ml de composition tombent dans une éprouvette ayant un diamètre de 50 mm et contenant 50 ml de la composition à tester. La mesure est faite 5 minutes après l'arrêt de l'écoulement de la composition.

Ces compositions peuvent se présenter sous la forme de liquides plus ou moins epaissis, de crèmes ou de gels et elles conviennent principalement au lavage et au soin des cheveux.

Lorsque les compositions conformes à l'invention sont mises en oeuvre comme des shampooings, elles sont simplement appliquées sur cheveux mouillés et la mousse générée par massage ou friction avec les mains est ensuite éliminée, après un éventuel temps de pause, par rinçage à l'eau, l'operation pouvant être répétée une ou plusieurs fois.

L'invention a également pour objet un procédé de lavage et de conditionnement des matières kératiniques telles que notamment les cheveux consistant à appliquer sur lesdites matières mouillées une quantité efficace d'une composition telle que définie ci-dessus, puis à effectuer un rinçage à l'eau après un éventuel temps de pause.

Les compositions conformes à l'invention sont également utilisables comme gels douche, bains moussants, comme produits démaquillants moussants, pour le lavage et le conditionnement des cheveux et/ou de la peau, auquel cas ils sont appliqués sur la peau et/ou les cheveux humides et sont rincés après application.

Des exemples concrets, mais nullement limitatifs, illustrant l'invention vont maintenant être donnés.

### EXEMPLE 1:

On a réalisé deux compositions de shampooings, l'une conforme à l'invention (composition A) et l'autre comparative (composition B) (MA signifie Matière Active):

| | A | B |
|---|---|---|
| Alkyl (C₈/C₁₀/C₁₂/C₁₄) polyglycoside (1,4) en solution aqueuse à 53%.(APG) (PLANTACARE 2000 de HENKEL) | 5,7 g MA | 3,5 g MA |
| Lauryléther sulfate de sodium oxyéthyléné à 2,2 moles d'oxyde d'éthylène en solution aqueuse à 70% de MA | 11,5 g MA | 13,7 g MA |
| Huile d'avocat | 6 g | 6 g |
| Conservateurs | qs | qs |
| Acide citrique, 1H₂O qs | pH 5,5 | pH 5,5 |
| Eau déminéralisée qsp | 100 g | 100 g |

Dans la composition A, le rapport tensioactif de type sulfate / APG est égal à 2. Dans la composition B (comparatif), le rapport tensioactif de type sulfate / APG est égal à 4.
Dans les deux compositions, la quantité totale de tensioactifs est identique: 17,2 g.

On a effectué un shampooing en appliquant environ 12 g de la composition A sur des cheveux sensibilisés préalablement mouillés. On fait mousser le shampooing puis on rince abondamment à l'eau.
On procède selon le même mode opératoire que ci-dessus avec la composition comparative B. Un panel d'experts a évalué la mousse des deux shampooings.
Le démarrage de la mousse est plus rapide avec la composition selon l'invention (A). La quantité de mousse développée lors de l'application est plus importante avec la composition A et la mousse a plus de consistance.
Un panel d'experts a évalué l'aspect des cheveux séchés.

Tous les experts indiquent que les cheveux traités avec la composition A selon l'invention sont plus souples, plus brillants et plus disciplinés que les cheveux traités avec la composition B.

### EXEMPLE 2 :

On a préparé une composition de shampooing selon l'invention de composition suivante (MA signifie Matière Active) :

| | |
|---|---|
| Alkyl (C₈/C₁₀/C₁₂/C₁₄) polyglycoside (1,4) en solution aqueuse à 53% de MA (PLANTACARE 2000 de HENKEL) | 7,4 g MA |
| Lauryléther sulfate de sodium oxyéthyléné à 2,2 moles d'oxyde d'éthylène en solution aqueuse à 70% de MA | 9,8 g MA |
| Huile d'avocat | 6 g |
| Conservateurs | qs |
| Acide citrique, 1H₂O qs | pH 5,5 |
| Eau déminéralisée qsp | 100 g |

Des cheveux ont été lavés à l'aide de cette composition. Ils ont ensuite été rincés à l'eau courante.
Avant le séchage, on constate que les cheveux sont très doux à l'état mouillé et se démêlent bien. Après séchage, on constate que les cheveux sont lisses, doux et brillants.

### EXEMPLE 3 :

On a préparé une composition de shampooing selon l'invention de composition suivante (MA signifie Matière Active) :

| | |
|---|---|
| Alkyl (C₈/C₁₀/C₁₂/C₁₄) polyglycoside (1,4) en solution aqueuse à 53% de MA (PLANTACARE 2000 de HENKEL) | 7,4 g MA |
| Lauryléther sulfate de sodium oxyéthyléné à 2,2 moles d'oxyde d'éthylène en solution aqueuse à 70% de MA | 9,8 g MA |
| Huile de jojoba | 6 g |
| Conservateurs | qs |
| Acide citrique, 1H₂O qs | pH 5,5 |
| Eau déminéralisée qsp | 100 g |

Des cheveux ont été lavés à l'aide de cette composition. Ils ont ensuite été rincés à l'eau courante.
Avant le séchage, on constate que les cheveux sont très doux à l'état mouillé et se démêlent bien. Après séchage, on constate que les cheveux sont lisses, doux et brillants.

### EXEMPLE 4:

On a préparé une composition de shampooing selon l'invention de composition suivante (MA signifie Matière Active) :

| | |
|---|---|
| Alkyl (C₈/C₁₀/C₁₂/C₁₄) polyglycoside (1,4) en solution aqueuse à 53% de MA (PLANTACARE 2000 de HENKEL) | 7,4 g MA |
| Lauryléther sulfate de sodium oxyéthyléné à 2,2 moles d'oxyde d'éthylène en solution aqueuse à 70% de MA | 9,8 g MA |
| Cocoylbétaïne en solution aqueuse à 30% de MA | 2,1 g MA |
| Huile d'avocat | 6 g |
| Mélange d'alcool cétylique et de 1-(hexadécyloxy)-2-octadécanol | 2 g |
| Monoisopropanolamide d'acides de coprah | 0,8 g |
| Conservateurs, parfums | qs |
| Acide citrique, 1H₂O qs | pH 5,5 |
| Eau déminéralisée qsp | 100 g |

Des cheveux ont été lavés à l'aide de cette composition. Ils ont ensuite été rincés à l'eau courante.
Avant le séchage, on constate que les cheveux sont très doux à l'état mouillé et se démêlent bien. Après séchage, on constate que les cheveux sont lisses, doux et brillants.

### EXEMPLE 5 :

On a préparé une composition de gel douche selon l'invention de composition suivante (MA signifie Matière Active) :

| | |
|---|---|
| Alkyl (C₈/C₁₀/C₁₂/C₁₄) polyglycoside (1,4) en solution aqueuse à 53% de MA (PLANTACARE 2000 de HENKEL) | 8 g MA |
| Lauryléther sulfate de sodium oxyéthyléné à 2,2 moles d'oxyde d'éthylène en solution aqueuse à 70% de MA | 10 g MA |
| Cocoylbétaïne en solution aqueuse à 30% de MA | 5gMA |
| Huile d'avocat | 4 g |
| Hydroxyéthylcellulose réticulée par l'épichlorhydrine et quaternisée par la triméthylamine (JR 400 de UNION CARBIDE) | 0,3 g |
| Mélange d'alcool cétylique et de 1-(hexadécyloxy)-2-octadécanol | 2 g |
| Monoisopropanolamide d'acides de coprah | 0,8 g |
| Glycérine | 3 g |
| Conservateurs, parfums | qs |
| Acide citrique, 1H₂O qs | pH 5,5 |
| Eau déminéralisée qsp | 100 g |

La peau lavée avec ce gel-douche présente un film protecteur, elle est bien hydratée et est très douce.

## Revendications

1. Composition moussante conditionnante et détergente, **caractérisée par le fait qu'**elle comprend, dans un milieu aqueux :
(A) au moins une huile végétale non volatile, dans des proportions de 0,2 à 20% en poids par rapport au poids total de la composition,
(B) au moins un tensioactif anionique de type sulfate, dans des proportions de 1 à 20% en poids par rapport au poids total de la composition
(C) au moins un agent tensioactif non ionique de type alkylpolyglycoside,
le rapport en poids (B)/(C) étant inférieur ou égal à 2.

2. Composition selon la revendication 1, **caractérisée par le fait que** ladite huile végétale est choisie parmi les huiles de tournesol, d'avocat, de jojoba, de maïs, d'amande douce, de soja, de courge, de pépins de raisin, de sésame, de noisette, de palme, de ricin, de noix, de noix de cajou, de purcellin.

3. Composition selon la revendication 1, **caractérisée par le fait que** ladite huile végétale est issue de végétaux dicolylédones.

4. Composition selon l'une quelconque des revendications 1 a 3, **caractérisée par le fait que** ladite huile végétale est choisie parmi les huiles d'avocat et de jojoba.

5. Composition selon l'une quelconque des revendications 1 à 4, **caractérisée par le fait que** ledit agent tensioactif non ionique de type alkylpolyglycoside est un composé de formule (I) :
R₁-O-(R₂O)ₜ-(G)ᵥ (I)
dans laquelle R, représente un radical alkyle linéaire ou ramifié, saturé ou insaturé, comportant de 8 à 24 atomes de carbone, un radical alkylphényle dont le radical alkyle linéaire ou ramifié comporte de 8 à 24 atomes de carbone, R₂ représente un radical alkylène comportant de 2 a 4 atomes de carbone, G représente un sucre réduit comportant de 5 à 6 atomes de carbone, t designe une valeur allant de 0 a 10 et v désigne une valeur allant de 1 a 15.

6. Composition selon la revendication 5, **caractérisée par le fait que** dans la formule (I), R₁ désigne un radical alkyle saturé ou insaturé, linéaire ou ramifié comportant de 8 a 14 atomes de carbone, t prend la valeur 0, G désigne le glucose, v prend une valeur de 1 à 4.

7. Composition selon l'une quelconque des revendications 1 à 6, **caractérisée en ce que** le tensioactif anionique sulfate est choisi parmi les sels des alkylsulfates, alkyléthersulfates, alkylamidoéthersulfates, alkylaryléthersulfates ; le radical alkyle de tous ces différents composés comportant de préférence de 8 à 24 atomes de carbone, et le radical aryle designant de préférence un groupement phényle ou benzyle.

8. Composition selon la revendication 7, **caractérisée par le fait que** le tensioactif anionique sulfate est choisi parmi les sels d'alkylsulfates et d'alkyléthersulfates et leurs mélanges.

9. Composition selon l'une quelconque des revendications 1 à 8, **caractérisée par le fait que** la composition comprend au moins un agent tensioactif amphotère.

10. Composition selon la revendication 9, **caractérisée par le fait que** ledit agent tensioactif amphotère est choisi dans le groupe des bétaines.

11. Composition selon l'une quelconque des revendications 1 à 10, **caractérisée par le fait que** le rapport en poids (B)/(C) est compris entre 0,1 et 1,8.

12. Composition selon l'une quelconque des revendications 1 à 11, **caractérisée par le fait que** l'huile végétale non volatile est présante dans des concentrations comprises entre 0,2 et 10% en poids par rapport au poids total de la composition.

13. Composition selon l'une quelconque des revendications 1 à 12, **caractérisée par le fait que** le tensioactif anionique de type sulfate est present dans des concentrations comprises entre 3 et 20% en poids par rapport au poids total de la composition.

14. Composition selon l'une quelconque des revendications 1 à 13, **caractérisée par le fait que** ledit agent tensioactif non ionique de type alkylpolyglycoside est présent dans des concentrations pondérales comprises entre 0,5 et 15% par rapport au poids total de la composition.

15. Composition selon l'une quelconque des revendications 9 à 14, **caractérisée par le fait que** ledit agent tensioactif amphotère est présent dans des concentrations comprises entre 0,5 et 10% en poids par rapport au poids total de la composition.

16. Composition selon l'une quelconque des revendications 1 à 15, **caractérisée par le fait que** la totalité des tensioactifs détergents est comprise entre 3 et 50% en poids par rapport au poids total de la composition.

17. Composition selon l'une quelconque des revendications 1 à 16, **caractérisée par le fait qu**'elle contient en plus un ou plusieurs adjuvants choisis par les tensioactifs cationiques, les polymères anioniques ou non ioniques ou cationiques ou amphotères, les protéines, les céramides, les pseudocéramides, les hydroxyacides, les vitamines, le panthénol, les silicones volatiles ou non volatiles, solubles et insolubles dans le milieu, les agents hydratants, les agents antipelliculaires ou antiseborrhéiques, les filtres solaires, les agents anti radicaux fibres et leurs melanges.

18. Composition selon la revendication 17, **caractérisée par le** fait le polymère cationique est choisi parmi les dérivés d'éther de cellulose quaternaires, les homopolymères de sel de diallyldiméthylammonium et les copolymères de sel de diallyldiméthylammonium et d'acrylamide en particulier les chlorures, les polysaccharides cationiques, les homopolymères et les copolymères éventuellement réticulés de sel de (méth)acryloyloxyéthyltriméthylammonium, l'Hexadimethrine chloride.

19. Composition selon l'une quelconque des revendications 17 et 18, **caractérisée en ce que** le polymère cationique représente de 0,001 % à 10 % en poids, de préférence de 0,005 % à 5 % en poids, et encore plus préférentiellement de 0,01 % à 3 % en poids, du poids total de la composition.

20. Utilisation de la composition telle que définie à l'une quelconque des revendications 1 à 19 pour le soin et le lavage simultanés des matières kératiniques telles que les cheveux et la peau.

21. Procédé de lavage et de conditionnement des matières kératiniques telles que les cheveux consistant à appliquer sur lesdites matières mouillées une quantité efficace d'une composition telle que définie dans l'une quelconque des revendications 1 à 19, puis à effectuer un rinçage à l'eau après un éventuel temps de pause.

## Patentansprüche

1. Konditionierende und reinigende, schäumende Zusammensetzung, **dadurch gekennzeichnet, dass** sie in einem wässerigen Medium enthält:
(A) mindestens ein nicht flüchtiges pflanzliches Öl, in Anteilen von 0,2 bis 20 Gew.-%, bezogen auf das Gesamtgewicht der Zusammensetzung.
(B) mindestens einen anionischen grenzflächenaktiven Stoff vom Sulfattyp, in Anteilen von 1 bis 20 Gew.-%, bezogen auf das Gesamtgewicht der Zusammensetzung.
(C) mindestens einen nichtionischen grenzflächenaktiven Stoff vom Typ Alkylpolyglycosid,
wobei das Gewichtsverhältnis (B) / (C) 2 beträgt oder unter 2 liegt.

2. Zusammensetzung nach Anspruch 1, **dadurch gekennzeichnet, dass** das pflanzliche Öl unter Sonnenblumenöl, Avocadoöl, Jojo-baöl, Maisöl, Süßmandelöl, Sojaöl, Kürbiskernöl, Traubenkernöl, Sesamöl, Haselnussöl, Palmöl, Ricinusöl, Nussöl, Cashewnussöl und Purcellinöl ausgewählt ist.

3. Zusammensetzung nach Anspruch 1, **dadurch gekennzeichnet, dass** das pflanzliche Öl von Dikotyledonen stammt.

4. Zusammensetzung nach einem der Ansprüche 1 bis 3, **dadurch gekennzeichnet, dass** das pflanzliche Öl unter Avocadoöl und Jojobaöl ausgewählt.

5. Zusammensetzung nach einem der Ansprüche 1 bis 4, **dadurch gekennzeichnet, dass** das pflanzliche Öl, dass der nichtionische grenzflächenaktive Stoff vom Alkylpolyglycosidtyp eine Verbindung der folgenden Formel (I) ist:
R₁-O-(R₂O)ₜ-(G)ᵥ (I)
worin R₁ eine geradkettige oder verzweigte, gesättigte oder ungesättigte Alkylgruppe mit 8 bis 24 Kohlenstoffatomen oder eine Alkylphenylgruppe, deren geradkettige oder verzweigte Alkylgruppe 8 bis 24 Kohlenstoffatome enthält, bedeutet,
R₂ eine Alkylengruppe mit 2 bis 4 Kohlenstoffatomen ist,
G einen reduzierten Zucker mit 5 bis 6 Kohlenstoffatomen bedeutet,
t einen Wert von 0 bis 10 und
v einen Wert von 1 bis 15 aufweist.

6. Zusammensetzung nach Anspruch 5, **dadurch gekennzeichnet, dass** in der Formel (I) R₁ eine gesättigte oder ungesättigte, geradkettige oder verzweigte Alkylgruppe mit 8 bis 14 Kohlenstoffatomen bedeutet, t den Wert 0 hat, G Glucose bedeutet und v einen Wert von 1 bis 4 aufweist.

7. Zusammensetzung nach einem der Ansprüche 1 bis 6, **dadurch gekennzeichnet, dass** der anionische grenzflächenaktive Stoff vom Sulfattyp unter den Salzen von Alkylsulfaten, Alkylethersulfaten, Alkylamidoethersulfaten, Alkylarylethersulfaten ausgewählt ist, wobei die Alkylgruppe dieser verschiedenen Verbindungen vorzugsweise 8 bis 24 Kohlenstoffatome aufweist und die Arylgruppe vorzugsweise eine Phenylgruppe oder Benzylgruppe bedeutet.

8. Zusammensetzung nach Anspruch 7, **dadurch gekennzeichnet, dass** der anionische grenzflächenaktive Stoff vom Sulfattyp unter den Salzen von Alkylsulfaten und Alkylethersulfaten und deren Gemischen ausgewählt ist.

9. Zusammensetzung nach einem der Ansprüche 1 bis 8, **dadurch gekennzeichnet, dass** die Zusammensetzung mindestens einen amphoteren grenzflächenaktiven Stoff enthält.

10. Zusammensetzung nach Anspruch 9, **dadurch gekennzeichnet, dass** der amphotere grenzflächenaktive Stoff unter den Betainen ausgewählt.

11. Zusammensetzung nach einem der Ansprüche 1 bis 10, **dadurch gekennzeichnet, dass** das Gewichtsverhältnis (B)/(C) im Bereich von 0,1 bis 1,8 liegt.

12. Zusammensetzung nach einem der Ansprüche 1 bis 11, **dadurch gekennzeichnet, dass** das nicht flüchtige pflanzliche Öl in Konzentrationen von 0,2 bis 10 Gew.-%, bezogen auf das Gesamtgewicht der Zusammensetzung, enthalten ist.

13. Zusammensetzung nach einem der Ansprüche 1 bis 12, **dadurch gekennzeichnet, dass** der anionische grenzflächenaktive Stoff vom Sulfattyp in Konzentrationen von 3 bis 20 Gew.-%, bezogen auf das Gesamtgewicht der Zusammensetzung, enthalten ist.

14. Zusammensetzung nach einem der Ansprüche 1 bis 13, **dadurch gekennzeichnet, dass** der nichtionische grenzflächenaktive Stoff vom Alkylpolyglycosidtyp in Konzentrationen von 0,5 bis 15 Gew.-%, bezogen auf das Gesamtgewicht der Zusammensetzung, enthalten ist.

15. Zusammensetzung nach einem der Ansprüche 9 bis 14, **dadurch gekennzeichnet, dass** der amphotere grenzflächenaktive Stoff in Konzentrationen von 0,5 bis 10 Gew.-%, bezogen auf das Gesamtgewicht der Zusammensetzung, enthalten ist.

16. Zusammensetzung nach einem der Ansprüche 1 bis 15, **dadurch gekennzeichnet, dass** die reinigenden grenzflächenaktiven Stoffe insgesamt 3 bis 50 Gew.-%, bezogen auf das Gesamtgewicht der Zusammensetzung, ausmachen.

17. Zusammensetzung nach einem der Ansprüche 1 bis 16, **dadurch gekennzeichnet, dass** sie ferner einen oder mehrere Zusatzstoffe enthält, die unter den kationischen grenzflächenaktiven Stoffen, anionischen, nichtionischen, kationischen oder amphoteren Polymeren, Proteinen, Ceramiden, Pseudoceramiden, Hydroxysäuren, Vitaminen, Panthenol, flüchtigen oder nicht flüchtigen, in dem Medium löslichen oder unlöslichen Siliconen, Hydratisierungsmitteln, Wirkstoffen gegen Schuppen oder Seborrhoe, Sonnenschutzfiltern, Radikalfängern für freie Radikale und deren Gemischen ausgewählt sind.

18. Zusammensetzung nach Anspruch 17, **dadurch gekennzeichnet, dass** das kationische Polymer unter den quartáren Celluloseetherderivaten, Homopolymeren von Diallyldimethylammoniumsalzen und Copolymeren von Diallyldimethylammoniumsalzen und Acrylamid und insbesondere den Chloriden, kationischen Polysacchariden, gegebenenfalls vernetzten Homopolymeren und Copolymeren von (Meth)acryloyloxytrimethylammoniumsalzen und Hexadimethrine Chloride ausgewählt ist.

19. Zusammensetzung nach einem der Anspruüche 17 und 18, **dadurch gekennzeichnet, dass** das kationische Polymer 0,001 bis 10 Gew.-%, vorzugsweise 0,005 bis 5 Gew.-% und noch bevorzugter 0,01 bis 3 Gew.-% des Gesamtgewichts der Zusammensetzung ausmacht.

20. Verwendung der Zusammensetzung nach einem der Ansprüche 1 bis 19 für die Pflege und das gleichzeitige Reinigen von Keratinsubstanzen, wie Haaren und der Haut.

21. Verfahren zur Reinigung und zum Konditionieren von Keratinsubstanzen, wie Haaren, das darin besteht, auf die feuchten Keratinsubstanzen eine wirksame Menge einer Zusammensetzung nach einem der Ansprüche 1 bis 19 aufzubringen und gegebenenfalls nach einer Einwirkzeit mit Wasser zu spülen.

## Claims

1. Conditioning and detergent foaming composition, **characterized in that** it comprises, in an aqueous médium:
(A) at least one non-volatile vegetable oil, from 0.2 to 20% by weight with respect to the total weight of the composition,
(B) at least one anionic surfactant of sulphate type, from 1 to 20% by weight with respect to the total weight of the composition,
(C) at least one non-ionic surface-active agent of alkyl polyglycoside type,
the (B)/(C) ratio by weight being less than or equal to 2.

2. Composition according to Claim 1, **characterized in that** the said vegetable oil is chosen from sunflower oil, avocado oil, jojoba oil, maize oil, sweet almond oil, soybean oil, cucumber oil, grape seed oil, sesame oil, hazelnut oil, palm oil, castor oil, walnut oil, cashew nut oil or purcellin oil.

3. Composition according to Claim 1, **characterized in that** the said vegetable oil results from dicotyledonous plants.

4. Composition according to any one of Claims 1 to 3, **characterized in that** the said vegetable oil is chosen from avocado oil and jojoba oil.

5. Composition according to any one of Claims 1 to 4, **characterized in that** the said non-ionic surface-active agent of alkyl polyglycoside type is a compound of formula (I):
R₁-O-(R₂O)ₜ-(G)ᵥ (I)
in which R₁ represents a saturated or unsaturated, linear or branched alkyl radical comprising from 8 to 24 carbon atoms or an alkylphenyl radical in which the linear or branched alkyl radical comprises from 8 to 24 carbon atoms,
R₂ represents an alkylene radical comprising from 2 to 4 carbon atoms,
G represents a reduced sugar comprising from 5 to 6 carbon atoms,
t denotes a value ranging from 0 to 10 and
v denotes a value ranging from 1 to 15.

6. Composition according to Claim 5, **characterized in that**, in the formula (I), R₁ denotes a saturated or unsaturated, linear or branched alkyl radical comprising from 8 to 14 carbon atoms, t takes the value 0, G denotes glucose and v takes a value from 1 to 4.

7. Composition according to any one of Claims 1 to 6, **characterized in that** the sulphate anionic surfactant is chosen from the salts of alkyl sulphates, alkyl ether sulphates, alkylamido ether sulphates or alkylaryl ether sulphates; the alkyl radical of all these various compounds preferably comprising from 8 to 24 carbon atoms and the aryl radical preferably denoting a phenyl or benzyl group.

8. Composition according to Claim 7, **characterized in that** the sulphate anionic surfactant is chosen from the salts of alkyl sulphates and of alkyl ether sulphates and their mixtures.

9. Composition according to any one of Claims 1 to 8, **characterized in that** the composition comprises at least one amphoteric surface-active agent.

10. Composition according to Claim 9, **characterized in that** the said amphoteric surface-active agent is chosen from the group of the betaines.

11. Composition according to any one of Claims 1 to 10, **characterized in that** the (B)/(C) ratio by weight is between 0.1 and 1.8.

12. Composition according to any one of Claims 1 to 11, **characterized in that** the non-volatile vegetable oil is present in concentrations of between 0.2 and 10% by weight with respect to the total weight of the composition.

13. Composition according to any one of Claims 1 to 12, **characterized in that** the anionic surfactant of sulphate type is present in concentrations of between 3 and 20% by weight with respect to the total weight of the composition.

14. Composition according to any one of Claims 1 to 13, **characterized in that** the said non-ionic surface-active agent of alkyl polyglycoside type is present in concentrations by weight of between 0.5 and 15% with respect to the total weight of the composition.

15. Composition according to any one of Claims 9 to 14, **characterized in that** the said amphoteric surface-active agent is present in concentrations of between 0.5 and 10% by weight with respect to the total weight of the composition.

16. Composition according to any one of Claims 1 to 15, **characterized in that** the combined detergent surfactants are between 3 and 50% by weight with respect to the total weight of the composition.

17. Composition according to any one of Claims 1 to 16, **characterized in that** it additionally comprises one or more adjuvants chosen from cationic surfactants, anionic or non-ionic or cationic or amphoteric polymers, proteins, ceramides, pseudoceramides, hydroxy acids, vitamins, panthenol, volatile or non-volatile silicones which are soluble and insoluble in the medium, moisturizing agents, antidandruff or antiseborrhoeic agents, sunscreening agents, agents for combating free radicals and their mixtures.

18. Composition according to Claim 17, **characterized in that** the cationic polymer is chosen from quaternary cellulose ether derivatives, diallyldimethylammonium salt homopolymers and diallyldimethylammonium salt and acrylamide copolymers, in particular the chlorides, cationic polysaccharides, optionally crosslinked homopolymers and copolymers of (meth)acryloyloxyethyltrimethylammonium salt, or hexadimethrine chloride.

19. Composition according to either one of Claims 17 and 18, **characterized in that** the cationic polymer represents from 0.001% to 10% by weight, preferably from 0.005% to 5% by weight and more preferably still from 0.01% to 3% by weight of the total weight of the composition.

20. Use of the composition as defined in any one of Claims 1 to 19 for simultaneously caring for and washing keratinous substances, such as the hair and the skin.

21. Process for washing and conditioning keratinous substances, such as the hair, which consists in applying, to the said wet substances, an effective amount of a composition as defined in any one of Claims 1 to 19 and in then rinsing with water, after an optional resting time.
